# EUROPEAN PATENT SPECIFICATION

(11) **EP 1 554 316 B1**
(45) Date of publication and mention of the grant of the patent: **16.07.2008**
(21) Application number: 03735732.4
(22) Date of filing: 24.06.2003
(51) Int. Cl.: C08B 37/16, A61K 31/724, A61K 31/34

(54) **LIGNAN COMPLEXES**
LIGNANKOMPLEXE
COMPLEXES DE LIGNANES

(30) Priority: 29.08.2002 FI 20021545
(43) Date of publication of application: 20.07.2005
(73) Proprietor: Hormos Medical Ltd., 20520 Turku (FI)
(72) Inventor: JÄRVINEN, Tomi, FIN-70200 Kuopio (FI); JARHO, Pekka, FIN-70200 Kuopio (FI); UNKILA, Mikko, FIN-21500 PIIKKIÖ (FI); HIILOVAARA-TEIJO, Mervi, FIN-21870 Riihikoski (FI)
(74) Representative: Nylund, Solveig Helena
(86) International application number: PCT/FI2003/000511
(87) International publication number: WO 2004/020474

(56) References cited:
- EP-A2- 0 387 000
- VINCIERI FRANCO F. ET AL.: 'Permeation of oenantie aquatica fruit tincture-beta-cyclodextrin complex through artificial membranes' IL FARMACO vol. 49, no. 1, 1994, pages 63 - 67, XP002969239
- VAN UDEN WIM ET AL.: 'The large-scale isolation of deoxypodophyllotoxin from rhizomes of anthriscus sylvestris followed by its bioconversion into 5-methoxypodophyllotoxin beta-D-glucoside by cell cultures of linum flavum' J. NAT. PROD. vol. 60, 1997, pages 401 - 403, XP002969240

## Description

This invention relates to cyclodextrin complexes of hydroxymatairesinol or its esters, and to the use of such complexes in various food compositions, dietary supplement products or pharmaceuticals.

### BACKGROUND OF THE INVENTION

Cyclodextrins and their use:

Cyclodextrins (CDs) are a group of cyclic oligosaccharides which have been shown to improve pharmaceutical properties of lipophilic drugs by forming inclusion complexes (Frömming K-H, Szejtli J, Cyclodextrins in pharmacy, Kluwer Academic Publishers, Dordrecht, 1994). Cyclodextrins are cone-shaped molecules with two openings. The cavity of the molecule is hydrophobic while the surface of the molecule is hydrophilic. An inclusion complex is formed when the lipophilic guest molecule, or part of it, enters into the apolar cavity of the cyclodextrin. Inclusion complex formation is mainly based on hydrophobic interactions between drug and cyclodextrin, and no covalent bonds are formed during the complexation.

Cyclodextrins are either natural cyclodextrins or derivatives thereof (Thompson D: Cyclodextrins-enabling excipients: their present and future use in pharmaceuticals. Crit. Rev. Ther. Drug Carrier Syst. 14: 1 - 104, 1997).

Natural cyclodextrins are enzymatic degradation products of starch, formed from six (α-cyclodextrin or α-CD), seven (β-cyclodextrin or β-CD) or eight (γ-cyclodextrin or γ-CD) glucopyranose units. Modified cyclodextrins, such as methyl-, hydroxyalkyl-, and sulfoalkylether derivatives of natural cyclodextrins, have been developed to increase the aqueous solubility and pharmaceutical usefulness of natural cyclodextrins. So far, the most commonly studied cyclodextrin derivative in drug development is hydroxypropyl-β-cyclodextrin (HP-β-CD).

Cyclodextrins have traditionally been used to increase the aqueous solubility and chemical/physical stability of lipophilic drugs (Loftsson T, Brewster M E: Pharmaceutical applications of cyclodextrins. 1. drug solubilization and stabilization. J. Pharm. Sci. 85: 1017 -1025, 1996). However, the complexation of a drug with cyclodextrins may also increase its bioavailability or decrease side-effects (Rajewski R A, Stella V J: Pharmaceutical applications of cyclodextrins 2. in vivo drug delivery. J. Pharm. Sci. 85: 1142 -1169, 1996). In addition (as with food and cosmetics preparations), cyclodextrins have also been studied in drug formulations to mask the unpleasant taste or odour of drugs (Frömming and Szejtli 1994). Until now, the utilization of cyclodextrins has been limited to relative small molecules, but cyclodextrins are also useful with macromolecules (e.g., proteins and peptides) which will extend their utilization in the future (Irie T, Uekama K: Cyclodextrins in peptide and protein delivery. Adv. Drug Del. Rev. 36: 101-123, 1999).

A problem with natural β-CD is that it causes nephrotoxicity after parenteral administration (Irie T, Uekama K: Pharmaceutical applications of cyclodextrins . III. Toxicological issues and safety evaluation. J. Pharm. Sci. 86:147 -162, 1997). However, in oral administration β-CD does not show any toxicity due to its minor absorption from the gastrointestinal tract. Similarly, the other natural cyclodextrins and derivatives thereof do not absorb from the gastrointestinal tract due to the bulky and hydrophilic character of cyclodextrin molecules. In the gastrointestinal tract, cyclodextrins (except for natural γ-CD) are remarkably resistant to the usual starch hydrolysing enzymes. The cyclodextrins cannot be hydrolyzed by β-amylase and they are hydrolysed by α-amylase at a very low rate. The fundamental physiological difference between cyclodextrins and starch is that the metabolism of cyclodextrins takes place in the colon while starch is metabolized in the small intestine. The metabolites of cyclodextrins (maltose, glucose, acyclic maltodextrins) are rapidly metabolized further and finally excreted as CO₂ and H₂O. In general, introduction of substituents on the hydroxyl groups slows down enzymatic hydrolysis of the cyclodextrin by lowering its enzyme affinity.

### Lignans:

Lignans are phenolic compounds widely distributed in plants. They can be found in different parts (roots, leafs, stem, seeds, fruits) but mainly in small amounts. In many sources (seeds, fruits), lignans are found as glycosidic conjugates associated with fiber component of plants. The most common dietary source of mammalian lignan precursors are unrefined grain products. The highest concentrations in edible plants have been found in flaxseed, followed by unrefined grain products, particularly rye.

Considerable amounts of lignans are also found in coniferous trees. The type of lignans differs among different tree species and the amounts of lignans varies between different parts of the tree. The typical lignans in heartwood of Norway spruce (Picea abies) are hydroxymatairesinol (HMR), alpha-conidendrin, alpha-conidendric acid, matairesinol, isolariciresinol, secoisolariciresinol, liovil, picearesinol, lariciresinol and pinoresinol (Ekman R: Distribution of lignans in Norway spruce. Acta Academiae Aboensis, Ser B, 39:1-6, 1979). The far most abundant single component of lignans in spruce is HMR, about 60 per cent of total lignans, which occurs mainly in non-glycosylated form.

Plant lignans such as HMR matairesinol, lariciresinol and secoisolariciresinol, are converted by gut microflora to mammalian lignans, enterolactone or enterodiol. The mammalian lignans can also be manufactured synthetically (MB Groen and J Leemhius, Tetrahedron Letters 21, 5043, 1980).

Lignans are known to possess beneficial effects on human health. The health benefits obtained with lignan rich diet are, for example, decreased risk for various cancers and cardiovascular diseases (Adlercreutz (1998) Phytoestrogens and human health, In: Reproductive and Developmental Toxicology (edited by Korach, K.). pp. 299-371, Marcel & Dekker, NY.).

Lignans, such as HMR, WO 00/59946, have also been reported to inhibit lipid peroxidation and LDL oxidation and thus be useful as antioxidants.

Also lignans other than HMR have powerful antioxidant and antiinflammatory potential. The antioxidant action involves all the major free radicals such as superoxide anions and peroxyl radicals (K Prasad: Antioxidant activity of secoisolariciresinol diglucoside-derived metabolites, secoisolariciresinol, enterodiol and enterolactone. Int J Angiology 9:220-225 (2000)).

There is evidence to suggest that lignans may also prevent skin cancers (Thompson L.U. (1993): Potential health benefits and problems associated with antinutrients in foods. Food Res. Int. 26, 131-149).

While literature discloses several different chemical substances that can be complexed with different cyclodextrins, the cyclodextrin complexes of lignans or their derivatives have not been reported so far.

### OBJECTS AND SUMMARY OF THE INVENTION

There is a great need to provide novel improved formulations containing lignans or derivatives thereof for use as various kinds of food products, dietary supplements or pharmaceutical use in which formulations the solubility, bioavailability and stability of the active compound is satisfactory. Furthermore, masking of possible unpleasant taste or odour of the active compounds is also important.

Thus, according to one aspect, this invention concerns an inclusion complex of a lignan or lignan ester with a cyclodextrin, wherein the lignan is hydroxymatairesinol or a geometric isomer or a stereoisomer thereof, and the lignan ester is an ester of said hydroxymatairesinol, where one or both of the phenolic hydroxyl groups of hydroxymatairesinol are replaced by R'-CO-O- or R'-SO₂-O-, where R' is a C₁ to C₂₂ alkyl, alkenyl, arylalkyl, aralkenyl, or an aromatic group, and R' is unsubstituted or substituted with one or more hydroxy groups and/or one or more carboxyl groups, an oxo group or an amino group.

According to another aspect, this invention concerns a food product comprising said inclusion complex and a foodstuff.

According to a third aspect, the invention concerns a dietary supplement composition comprising said inclusion complex and an acceptable carrier.

According to a fourth aspect, the invention concerns a pharmaceutical composition comprising said inclusion complex and an acceptable carrier.

### BRIEF DESCRIPTION OF THE DRAWINGS

Figure 1 shows an example of B-type phase-solubility diagram, where the concentration of the active compound is shown on the y-axis and cyclodextrin concentration on the x-axis.
Figure 2 shows a phase-solubility diagram ofhydroxymatairesinol (HMR) with γ-cyclodextrin (γ-CD)
Figure 3 shows a phase-solubility diagram of hydroxymatairesinol diacetate (HMRdiAc) with γ-cyclodextrin
Figure 4 hows a phase-solubility diagram of hydroxymatairesinol (HMR) with hydroxypropyl-β-cyclodextrin (HP-β-CD)
Figure 5 shows the degradation of hydroxymatairesinol as function of time in the presence (squares) or absence (triangles) of 2 % γ-cyclodextrin.

### DETAILED DESCRIPTION OF THE INVENTION

### Preferred cyclodextrins:

Although any natural cyclodextrin or derivative thereof could be employed in this invention, natural α-, β- or γ-cyclodextrins are preferred. Particularly preferred is γ-cyclodextrin. Preferred derivatives are methyl-, hydroxyalkyl- and sulfoalkylether derivatives of natural cyclodextrins. An especially preferred cyclodextrin derivative is hydroxypropyl-β-cyclodextrin.

Scheme 1 shows the structure of hydroxymatairesinol.

"Esters" of lignans shall mean either phenolic esters (where the hydroxy groups in the phenol are esterified) or esters where hydroxy substituents in the lignan skeleton are esterified. Many esters of the latter kind are disclosed in the art. Certain phenolic lignan esters are also known in the art, namely the dibenzoate and the p-nitrodibenzoate of matairesinol; enterolactone diacetate; monoacetate, triacetate, p-hydroxymonobenzoate, and p-hydroxy-m-methoxymonobenzoate of hydroxymatairesionol; and tetraacetate and tetrabenzoate of secoisolariciresinol.

The ester is a phenolic ester, in particular a phenolic diester.

Preferable diphenolic lignan esters are, for example, esters of mono- or dicarboxylic fatty acids, hydroxy acids and sulfonic acids. As examples of suitable dicarboxylic acid lignan esters can be mentioned succinates, glutarates, and malonic acid esters. Lactic acid esters are examples of esters with hydroxysubstituted acids. Tartaric acid and citric acid esters are examples of esters of acids with several carboxylic groups and one or more hydroxy groups.

### Preparation of the inclusion complex:

The cyclodextrin inclusion complex of hydroxymatairesinol or its esters are preferably prepared by adding the compound to the cyclodextrin in an acetate buffer at pH 5. The complex formed can be precipitated and isolated.

However, the solid inclusion complex of lignan and cyclodextrin can also be prepared simply by freeze-drying or spray-drying the solution. In addition, methods such as kneading, grinding, neutralization and so-called slurry methods have been used to prepare solid inclusion complexes.

The inclusion complex according to this invention can be provided in the form of a pharmaceutical preparation, dietary supplement, or a food product.

The pharmaceutical preparation is preferably an oral formulation. The required amount of the active compound or mixture of compounds will vary with the compound and the particular condition to be prevented. A typical dose ranges from 1 to 2000 mg (calculated as lignan) per day and adult person, preferably 10 to 600 mg per day and adult person. Typical dosage forms include, but are not limited to, oral dosage forms such as powders, granules, capsules, tablets, caplets, lozenges, liquids, elixirs, emulsions and suspensions. All such dosage forms may include conventional carriers, diluents, excipients, binders and additives known to those skilled in the medicinal and pharmaceutical arts.

The carriers typically employed for the pharmaceutical composition or dietary supplement composition may be solid or liquid. Thus, for example, solid carriers include polysaccarides such as lactose, sucrose, gelatin, agar, while liquid carriers include aqueous solutions of salts, polysaccarides, complexing agents, surfactants, syrups, vegetable oils such as peanut oil or olive oil, and certain alcohols. However, any acceptable solid or liquid carrier can be used in the pharmaceutical preparation or other dietary or nutrition formula to be administered according to this invention.

A typical food product, suitable for use in the methods according to this invention, is especially a functional food, a nutritional supplement, a nutrient, a pharmafood, a nutraceutical, a clinical nutritional product, a health food, a designer food or any food product. The term food product shall also be understood to cover groceries and foodstuffs such as flour, other ingredients, certain liquids. A suitable concentration of the active compound in the food product is, for example, 1 to 1000 mg of active compound per 100 g of food product, preferably 10 to 100 mg of active compound per 100 g of food product.

The invention will be illuminated by the following non-restrictive Experimental Section.

### EXPERIMENTAL SECTION

### Materials and methods

### Chemicals

The lignans and lignan esters (hydroxymatairesinol (HMR) and hydroxymatairesinol diacetate (HMRdiAc), were received from Hormos Nutraceutical Ltd. and natural γ-CD and HP-β-CD was purchased from Wacker-Chemie GmbH (Burghausen, Germany). All other chemicals used were of analytical grade.

### Apparatus

The samples from the solubility and stability studies were analysed by using the HPLC system which consist of the UV-detector (L-7400), interface module (D-7000), pump (L-7100) and autosampler (L-7250, Merck Hitachi, Japan). Purospher^{®} reversed phase column (RP-18e, 5 µm, 125 x 4 mm) was used in all chromatographic separations.

### Solubility studies

The complexation of lignans and lignan esters with γ-CD was studied by using the phase-solubility method of Higuchi and Connors (Higuchi T, Connors K.A. Phase-solubility techniques. Adv. Anal. Chem. Instr. 4: 117-212, 1965). An excess amount of lignan or lignan ester was added to acetate buffer (0.16 M; pH 5.0; ionic strength 0.5) containing various concentrations of γ-CD (0 -10 %). The suspensions were shaken in the dark (25 °C) for 24 hour and the pH of the suspensions were monitored during the equilibration. The pH of suspensions was adjusted to 5.0 with HCl or NaOH if necessary. After equilibration, the suspensions were filtered through 0.45 µm membrane filters and analysed by HPLC.

The phase solubility studies with HMR was also performed with HP-β-CD (0 -10 %). In the case of EP-β-CD the equilibration time was 72 hours.

### Stability studies

The chemical stability of HMR was studied in acetate buffer (0.16 M; pH 5.0; ionic strength 0.5) in the presence and absence of 2 % γ-CD at 30 °C. All the solutions were prepared by dissolving 1.5 - 2.0 mg of HMR into 20 ml of the solutions mentioned above, and the concentration of the remaining HMR was determined at appropriate intervals by HPLC. The pseudo-first order rate constant for overall degradation of HMR was determined from the slopes of the linear semilogarithmic plots of remaining HMR versus time. The results of the stability studies were calculated as an average of three determinations.

### Results

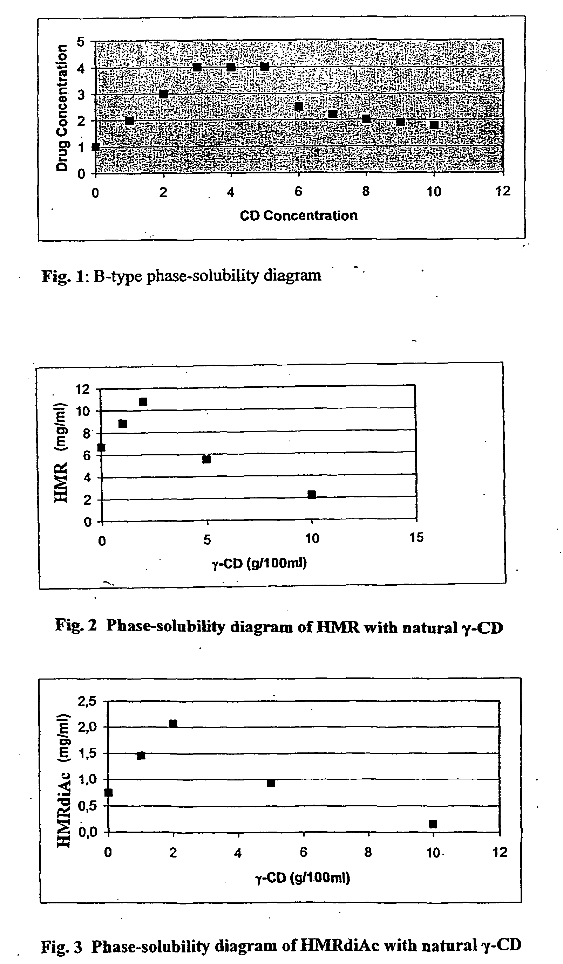

Figure 1 shows an example of the B-type phase-solubility diagram. In B-type phase-solubility diagram the concentration of the active compound, e.g. the complexed drug, first increases with increasing cyclodextrin concentration due to complexation of the active compound with the cyclodextrin molecules. However, after initial improvement in compound solubility, the maximum solubility of the complex is achieved and no further improvement is reached with increasing cyclodextrin concentration (highest part of the diagram). At a certain cyclodextrin concentration, the solubility of the compound begins to decrease in the B-type phase-solubility diagram, because at high cyclodextrin concentrations the compound forms lower solubility complexes with cyclodextrins. The B-type phase-solubility behaviour is typical for natural cyclodextrins and has been described earlier e.g. with steroid hormones (Uekama K, Fujinaga T, Hirayama F, Otagiri M, Yamasaki M. Inclusion complexation of steroid hormones with cyclodextrins in water and in solid phase. Int. J. Pharm. 10: 1-15, 1982).

### Solubility studies

Table 1 shows the effect of γ-CD on the aqueous solubility of hydroxymatairesinol and its diacetate at pH 5.0. The same data are also shown in figures 2-6 showing the phase-solubility data in graphical form. Figures 2-3 show that all the lignans and lignan esters studied form B-type phase solubility diagram with γ-CD.

**Table 1. The effect of γ-CD on the aqueous solubility of lignans (0.16 M acetate buffer; pH 5.0; µ = 0.5, 25 °C; equilibration time 24 hours)**

| γ-CD (g/100ml) | HMR (mg/ml) | HMRdiAc (mg/ml) |
|---|---|---|
| 0 | 6.70 | 0.75 |
| 1 | 8.85 | 1.46 |
| 2 | 10.80 | 2.07 |
| 5 | 5.50 | 0.94 |
| 10 | 2.21 | 0.16 |

| | | |
|---|---|---|
| *= concentration is under the detection limit of the HPLC method employed. | | |

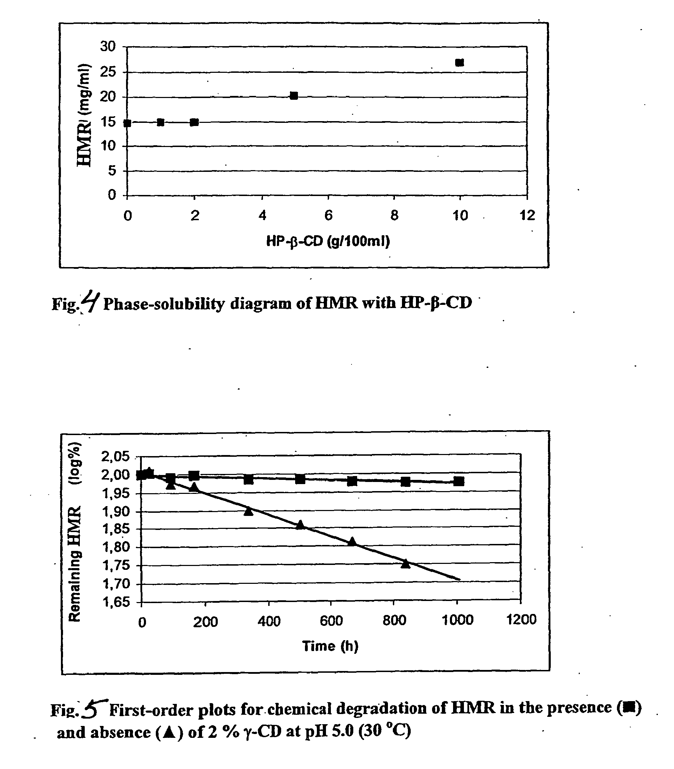

The complexation of HMR was also studied with HP-β-CD which is the most commonly used cyclodextrin derivative in drug development at present. The results (Table 2) show that HP-β-CD forms an inclusion complex with HMR and increases aqueous solubility, of HMR. The same data are also shown in Figure 4.

**Table 2. The effect of HP-β-CD on aqueous solubility of HMR (0.16 M acetate buffer; pH 5.0; µ = 0.5; 25 °C; equilibration time 72 hours)**

| HP-β-CD concentration (g/100 ml) | Aqueous solubility of HMR (mg/ml) |
|---|---|
| 0 | 14.56 |
| 1 | 14.90 |
| 2 | 14.94 |
| 5 | 20.16 |
| 10 | 26.83 |

### Stability studies

The overall degradation of HMR followed first-order kinetics in the presence and absence of 2 % γ-CD at pH 5.0 (Figure 8). Table 3 shows the first-order rate constants, half-lives (t_{1/2}) and shelf-lives (t_{90%}) for the chemical degradation of HMR.

The stability studies showed that 2 % of γ-CD increases the chemical stability of HMR about 12-fold at pH 5.0.

**Table 3. First-order rate constants (k_{obs}), half-lives (t_{1/2}) and shelf-lives (t_{90%}) for chemical degradation of HM-3000 at pH 5.0 (30 °C).**

| Vehicle | k_{obs}(h⁻¹) | t_{1/2}(h) | t_{90%}(h) |
|---|---|---|---|
| 0.16 M Acetate buffer (pH 5.0) | | | |
| - Without γ-CD | 6.91 x 10⁻⁴ | 1003 | 152 |
| - With 2% γ-CD | 5.71 x 10⁻⁵ | 12134 | 1845 |

### Conclusions

The results show that lignans and lignan esters form complexes with natural γ-cyclodextrin. With all the lignans and lignan esters studied, γ-CD complexation increased the aqueous solubility of the compounds at low γ-CD concentrations. However, at high γ-CD concentrations lignans and lignan esters form higher order complexes with natural γ-CD which results in decreased aqueous solubility (B-type phase-solubility behaviour). The main advantage of the B-type phase-solubility behaviour is the simple and effective preparation of the pure inclusion complexes by precipitation. Usually CD-containing products are the mixtures of non-complexed molecules of the active agent, complexed molecules of the active agent, and "empty" CD molecules. However, the B-type phase solubility behaviour allows the preparation of pure cyclodextrin complexes of the active agent, i.e. no free cyclodextrin molecules and molecules of the active agent are present in the product.

The present study also shows that the complexation of HMR with γ-CD significantly increases the aqueous stability of HMR. The present study was carried out at 2 % γ-CD concentration where HMR has the best solubility and the stoichiometry of the complex is most probably 1:1. Thus, it might be possible to improve the aqueous stability of HMR further by increasing the CD concentration which also changes the stoichiometry of the complex.

In addition, the present study shows that HP-β-CD can be used to improve the aqueous solubility of HMR.

The present study shows that the solubility of HMR without CDs is highly dependent on the equilibration time. Thus, it is important to point out that one of the major benefits of cyclodextrin complexation of lignans or lignan esters may also be the significant improvement the dissolution rate of the compounds, because cyclodextrins have been shown to increase the dissolution rate of lipophilic compounds in various applications.

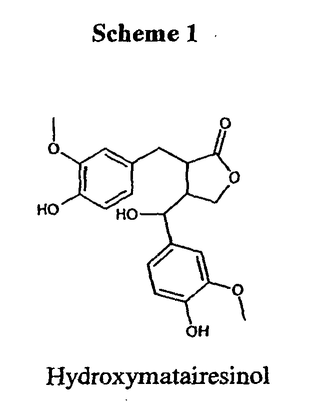

## Claims

1. An inclusion complex of a lignan or lignan ester with a cyclodextrin, wherein the lignan is hydroxymatairesinol or a geometric isomer or a stereoisomer thereof, and the lignan ester is an ester of said hydroxymatairesinol, where one or both of the phenolic hydroxyl groups of hydroxymatairesinol are replaced by R'-CO-O- or R'-SO₂-O-, where R' is a C₁ to C₂₂ alkyl, alkenyl, arylalkyl, aralkenyl, or an aromatic group, and R' is unsubstituted or substituted with one or more hydroxy groups and/or one or more carboxyl groups, an oxo group or an amino group.

2. The complex according to claim 1 wherein the cyclodextrin is α-cyclodextrin, β-cyclodextrin, γ-cyclodextrin or a derivative thereof.

3. The complex according to claim 2 wherein the cyclodextrin is natural cyclodextrin.

4. The complex according to claim 2 wherein the cyclodextrin is γ-cyclodextrin or hydroxypropyl-β-cyclodextrin.

5. The complex according to any of the claims 1-4, wherein both of the phenolic hydroxyl groups in hydroxymatairesinol are replaced by ester groups R'-CO-O- or R'-SO₂-O- and R' is as defined in claim 1.

6. A food product comprising a complex according to any of the claims 1-5 and a foodstuff.

7. The food product according to claim 6, which is a functional food, a nutritional supplement, a nutrient, a pharmafood, a nutraceutical, a clinical nutrition product, a health food or a designer food.

8. A dietary supplement composition comprising a complex according to any of the claims 1-5 and an acceptable carrier.

9. A pharmaceutical composition comprising a complex according to any of the claims 1-5 and an acceptable carrier.

## Patentansprüche

1. Einschlusskomplex eines Lignans oder Lignanesters mit einem Cyclodextrin, wobei das Lignan Hydroxymatairesinol oder ein geometrisches Isomer oder ein Stereoisomer davon ist, und der Lignanester ein Ester des Hydroxymatairesinols ist, wobei eine oder beide der phenolischen Hydroxygruppen des Hydroxymatairesinols durch R'-CO-O- oder R'-S0₂-O- ersetzt sind, wobei R' ein C₁-C₂₂-Alkyl, Alkenyl, Arylalkyl, Aralkenyl oder ein aromatischer Rest ist, und R' unsubstituiert oder substituiert mit einer oder mehreren Hydroxygruppen und/oder einer oder mehreren Carboxylgruppen, einer Oxogruppe oder einer Aminogruppe ist.

2. Komplex gemäß Anspruch 1, wobei das Cyclodextrin α-Cyclodextrin, β-Cyclodextrin, γ-Cyclodextrin oder ein Derivat davon ist.

3. Komplex gemäß Anspruch 2, wobei das Cyclodextrin natürliches Cyclodextrin ist.

4. Komplex gemäß Anspruch 2, wobei das Cyclodextrin γ-Cyclodextrin oder Hydroxypropyl-β-cyclodextrin ist.

5. Komplex gemäß einem der Ansprüche 1 bis 4, wobei beide phenolischen Hydroxygruppen des Hydroxymatairesinols durch Esterreste R'-CO-O- oder R'-SO₂-O-ersetzt sind und R' wie in Anspruch 1 definiert ist.

6. Lebensmittel, umfassend einen Komplex gemäß einem der Ansprüche 1 bis 5 und ein Nahrungsmittel.

7. Lebensmittel gemäß Anspruch 6, welches ein funktionelles Lebensmittel, ein Nahrungsergänzungsmittel, ein Nährstoff, ein Pharmafood, ein Nutraceutical, ein klinisches Nahrungserzeugnis, Reformkost oder Designerfood ist.

8. Nahrungsergänzungsmittelzusammensetzung, umfassend einen Komplex gemäß einem der Ansprüche 1 bis 5 und einen verträglichen Träger.

9. Arzneimittel, umfassend einen Komplex gemäß einem der Ansprüche 1 bis 5 und einen verträglichen Träger.

## Revendications

1. Complexe par inclusion d'un lignane ou d'un ester de lignane avec une cyclodextrine, dans lequel le lignane est l'hydroxymatairésinol ou un isomère géométrique ou un stéréoisomère de celui-ci, et l'ester de lignane est un ester dudit hydroxymatairésinol, où l'un ou les deux des groupes hydroxyle phénoliques de l'hydroxymatairésinol sont remplacés par R'-CO-O- ou R'-SO₂-O-, où R' est un groupe alkyle, alcényle, arylalkyle, aralcényle, en C₁ à C₂₂, ou un groupe aromatique, et R' est non substitué ou substitué par un ou plusieurs groupes hydroxy et/ou un ou plusieurs groupes carboxyle, un groupe oxo ou un groupe amino.

2. Complexe selon la revendication 1, dans lequel la cyclodextrine est une α-cyclodextrine, une β-cyclodextrine, une γ-cyclodextrine, ou un dérivé de celles-ci.

3. Complexe selon la revendication 2, dans lequel la cyclodextrine est une cyclodextrine naturelle.

4. Complexe selon la revendication 2, dans lequel la cyclodextrine est une γ-cyclodextrine ou une hydroxypropyl-β-cyclodextrine.

5. Complexe selon l'une quelconque des revendications 1 à 4, dans lequel les deux groupes hydroxyle phénoliques dans l'hydroxymatairésinol sont remplacés par des groupes esters R'-CO-O- ou R'-SO₂-O- et R' est tel que défini dans la revendication 1.

6. Produit alimentaire comprenant un complexe selon l'une quelconque des revendications 1 à 5 et une charge alimentaire.

7. Produit alimentaire selon la revendication 6, qui est un aliment fonctionnel, un complément nutritionnel, un nutriment, un alicament, un nutraceutique, un produit de nutrition clinique, un aliment de santé ou un aliment à usage médicinal spécifié.

8. Composition de complément alimentaire comprenant un complexe selon l'une quelconque des revendications 1 à 5 et un véhicule acceptable.

9. Composition pharmaceutique comprenant un complexe selon l'une quelconque des revendications 1 à 5 et un véhicule acceptable.
